# EUROPEAN PATENT SPECIFICATION

(11) **EP 1 574 201 B1**
(45) Date of publication and mention of the grant of the patent: **19.12.2007**
(21) Application number: 05003529.4
(22) Date of filing: 18.02.2005
(51) Int. Cl.: A61K 8/00, A61K 47/10

(54) **Composition comprising benzyloxyethanol as preservative**
Zusammensetzung enthaltend Benzyloxyethanol als Konservierungsmittel
Composition comprenant benzyloxyethanol comme conservateur

(30) Priority: 18.02.2004 JP 2004041840; 18.02.2004 JP 2004041841
(43) Date of publication of application: 14.09.2005
(73) Proprietor: KAO CORPORATION, Tokyo 103-8210 (JP)
(72) Inventor: Kubota, Hiromi, Ichikaimachi, Haga-gun Tochigi 321-3497 (JP); Tsugukuni, Takashi, Ichikaimachi, Haga-gun Tochigi 321-3497 (JP); Tokuda, Hajime, Ichikaimachi, Haga-gun Tochigi 321-3497 (JP); Inaba, Sayaka, Sumida-ku Tokyo 131-8501 (JP); Hosokawa, Masaru, Sumida-ku Tokyo 131-8501 (JP)
(74) Representative: HOFFMANN EITLE

(56) References cited:
- EP-A- 1 283 030
- EP-A- 1 479 368
- EP-A- 1 491 180
- US-A- 5 587 155

## Description

### Field of the Invention

The present invention relates to a composition of an external dermatological agent exhibiting antimicrobial activity or preservative efficacy, and to use of benzyloxyethanol.

### Background of the Invention

Conventional cosmetics and drugs for external-use (e.g., a cream, a lotion, an emulsion, a pack, and an ointment) contain a preservative or an antimicrobial agent, such as p-hydroxybenzoic acid ester or salt thereof, benzoic acid salt (e.g., sodium benzoate), dehydroacetic acid or salt thereof, quaternary ammonium salt (e.g., benzalkonium chloride), methylchloroisothiazolinone (Kathon CG), alcohol (e.g., ethanol or isopropanol), or triclosan. Incorporation of such preservatives or antimicrobial agents is for the purpose of preventing deterioration of such a cosmetics or drugs upon products or use thereof, which would otherwise occur as a result of contamination with microorganisms (e.g., bacteria or fungi), or for the purpose of suppressing growth of bacteria which cause, for example, acne.

However, such preservatives or antimicrobial agents have often been reported of skin irritation or skin sensitization. Therefore, in many cases, the formulation amount of such agents are limited from the viewpoint of safety, and the amount thereof required for attaining effective antimicrobial activity fails to be formulated. In view of the foregoing, demand has arisen for an antimicrobial substance exhibiting more excellent antimicrobial activity and high safety.

Benzyloxyethanol has activity of enhancing the rate of permeation of a hair treatment ingredient into hair, and thus is incorporated as a permeation enhancer into a hair treatment composition or a hair dye composition (Japanese Patent Nos. 1952826 and 1991404). However, benzyloxyethanol has not yet been known to exhibit antimicrobial activity.

EP 1 283 030 relates to a hair treatment composition, comprising a tertiary amine, which can impart fibre or hair with good suppleness and smoothness during wetting and after drying.

US 5 587 155 refers to a hair cosmetic composition which is excellent in hair-conditioning effects and which also can impart resilience to the hair, prevent damage to the hair and keep the waves or curls of the hair beautiful. Said hair cosmetic composition comprises (a) at least one compound selected from fatty acids containing a linear or branched alkyl or alkenyl group having 12-40 carbon atoms, salts thereof and fatty acid esters composed of one of the fatty acids and a polyhydric alcohol; (b) at least one aromatic alcohol represented by the general formula (1); and (c) at least one cationic surfactant.

EP 1 491 180 A1, representing prior art according to Article 54(3) EPC, relates to a preparation process for a hair cosmetic composition comprising an amphipatic amine lipid, a tertiary amine or quarternary ammonium salt, and a higher alcohol. Example 6 of this document discloses a specific composition of said type, which further comprises 0.5 wt% of benzyloxyethanol and 0.1 wt% of methylparaben.

### Summary of the Invention

The present invention provides a composition of an external dermatological agent containing benzyloxyethanol in an amount between 0.1 to 2 mass-% and a preservative, according to present claim 1.

The present invention also provides use of benzyloxyethanol for providing an object with antimicrobial and antifungal activities.

The present invention also relates to use of benzyloxyethanol for providing an object with preservative efficacy.

### Detailed Description of the Invention

The present invention provides a composition of an external dermatological agent exhibiting excellent antimicrobial activity and high safety.

The present inventors have searched for a substance which exhibits high safety and antimicrobial activity and which can be employed in cosmetics or drugs, and as a result have found that benzyloxyethanol exhibits excellent antimicrobial activity and can be employed as an antimicrobial and antifungal agent or a preservative. The present inventors have also found that when benzyloxyethanol is incorporated into a skin external composition together with a paraben as preservative, as compared with the case where benzyloxyethanol is employed solely, the resultant composition exhibits excellent antimicrobial or preservative activity and high safety; i.e., the composition is suitable for use in, for example, drugs or cosmetics.

The composition of an external dermatological agent of the present invention exhibits safety and excellent antimicrobial activity and preservative efficacy. Use of benzyloxyethanol can impart antimicrobial activity or preservative efficacy to, for example, drugs, consumer products, or miscellaneous products.

The composition of an external dermatological agent of the present invention is an external composition containing benzyloxyethanol and a preservative, which is a paraben, excluding the following composition:

| | | |
|---|---|---|
| N,N-Dimethyloctadecyloxypropylamine | | 2.4 mass-% |
| Stearyl alcohol | | 7.2 mass-% |
| Propylene glycol | | 2.0 mass-% |
| Amphiphatic amide lipid A | | 0.5 mass-% |
| Sunflower oil | | 0.9 mass-% |
| Camellia oil | | 0.2 mass-% |
| Cholesteryl hydroxystearate | | 0.1 mass-% |
| Dipentaerythrol fatty acid ester | | 0.3 mass-% |
| | (fatty acids: hydroxystearic acid, stearic acid and rosin acid) | |
| Benzyloxyethanol | | 0.5 mass-% |
| Methylpolysiloxane (100000 mPa*s) | | 3.7 mass-% |
| Aqueous solution of lactic acid (90 mass-%) | | 0.9 mass-% |
| Methylparaben | | 0.1 mass-% |
| Perfume | | 0.3 mass-% |
| Purified water | | balance. |

Specific examples of the product form of the composition include basic skin care products, make-up products, and various drugs (e.g., an ointment).

Benzyloxyethanol, which is a known substance, can be produced by means of a known technique. The benzyloxyethanol employed in the present invention may be a commercially available product, such as Benzyl Glycol (BzG) (product of Nippon Nyukazai Co., Ltd.).

The amount of benzyloxyethanol contained in the composition of an external dermatological agent of the present invention is 0.1 to 2 mass%.

The preservative employed in the present invention is a parabens. Parabens are used from the viewpoint of antimicrobial effects.These parabens may be used solely or in combination of two or more.

As described in the Examples section hereinbelow, benzyloxyethanol exhibits excellent antimicrobial activity against, for example, bacteria and fungi. When benzyloxyethanol is employed in combination with a paraben, by virtue of the synergistic effect of benzyloxyethanol and the paraben, the resulting antimicrobial activity is considerably enhanced as compared with the case where benzyloxyethanol or the paraben is employed singly. Therefore, when benzyloxyethanol and the paraben are employed in combination, the amount of benzyloxyethanol or the paraben can be considerably reduced as compared with the case where these are employed singly.

In general, the formulation amount of the paraben, which varies in accordance with the intended use of the composition, is regulated to 0.2 to 1.0 mass% in order to secure the preservative effect of the paraben. However, the amount of the paraben to be formulated can be reduced to 0.02 to 0.5 mass% (preferably 0.05 to 0.4 mass%); i.e., the amount of the paraben can be reduced by up to 99%. Therefore, the composition of an external dermatological agent of the present invention can be used safely even by a person who is sensitive to a paraben.

Examples of the paraben to be employed in the present invention include p-hydroxybenzoic acid esters and salts thereof. Specific examples include methyl paraben, ethyl paraben, propyl paraben, butyl paraben, and salts thereof.

The composition of an external dermatological agent of the present invention may appropriately contain, in accordance with its intended use, an ingredient such as a humectant, a UV protection agent, a vitamin, an animal or plant extracts, an anti-inflammatory agent, a whitening agent, a vasodilator, an astringent, a refreshing agent, a hormone drug, an anti-wrinkle agent, an analgesic agent, an antipruritic agent, a sterilizing agent, or a skin softening agent. As described above, the composition of an external dermatological agent of the present invention is employed in the form of, for example, cosmetics or drugs, Examples of the product form of the composition of an external dermatological agent include an aqueous solution, a solubilization system, an emulsion, a gel, a paste, an ointment, an aerosol, a water-oil two-phase system, and a water-oil-powder three-phase system. Specific examples of the product form of the cosmetics include a cream, a emulsion, a lotion, an essence, a sunscreen lipstick, a foundation, a mascara, an eyeliner, a pack, a mask, a bath agent, and a skin cleanser, and specific examples of the product form of the drugs include a variety of ointments.

If desired, the composition of an external dermatological agent of the present invention may contain a known base ingredient such as an oil ingredient, a higher fatty acid, an alcohol, a silicone, a surfactant, a water-soluble polymer, a chelating agent, a pH adjusting agent, a thickener, a powdery ingredient, a colorant, a perfume, or water, which is appropriately selected in accordance with the aforementioned product form of the composition.

As described above, benzyloxyethanol, when used alone, exhibits excellent antimicrobial activity against, for example, bacteria or fungi. Therefore, benzyloxyethanol itself can be employed as an antibacterial and antifungal agent or as a preservative for providing an object with antibacterial and antifungal activity or preservative efficacy. Specifically, benzyloxyethanol or a combination of benzyloxyethanol and paraben can be formulated in cosmetics, drugs, or a consumer antimicrobial product for the purpose of inhibiting growth of microorganisms. Alternatively, benzyloxyethanol or a combination of benzyloxyethanol and a paraben can be formulated in a cosmetics, a drugs, a consumer products, or a miscellaneous products for the purpose of preventing deterioration of the product by microorganisms.

Such a cosmetics or drugs can be produced by appropriately adding, to benzyloxyethanol, a solvent such as water, alcohol (e.g., ethyl alcohol or isopropyl alcohol), or glycerin, an oil ingredient, a higher fatty acid, an alcohol, a silicone, a surfactant, a water-soluble polymer, a chelating agent, a pH adjusting agent, a thickener, a powdery ingredient, a colorant, a perfume, a bactericide or a fungicide.

Examples of the form of the product include an aqueous solution, a solubilization system, an emulsion, a water-oil two-phase system, and a water-oil-powder three-phase system. Specific examples of the product form include an ointment, a cream, an emulsion, and a solution.

The amount of benzyloxyethanol to be incorporated into the aforementioned cosmetics or drugs varies in accordance with, for example, the intended use or form of the product, but is regulated to 0.1 to 2 mass%, much more preferably 0.1 to 1 mass%. When a paraben is employed in combination with benzyloxyethanol, the amount of the paraben to be incorporated into the aforementioned product is regulated to 0.02 to 0.5 mass%, preferably 0.05 to 0.4 mass%.

Examples of consumer products or miscellaneous products containing benzyloxyethanol or a combination of benzyloxyethanol and a paraben include antimicrobial products such as an antibacterial and antifungal agent, a liquid-type antimicrobial finishing agent for clothing, an antimicrobial detergent for clothing, an antimicrobial softening agent for clothing, an antimicrobial bleaching agent for clothing, an antimicrobial detergent for household use, an antimicrobial detergent for a drain outlet, an antimicrobial detergent for a bathroom, and an antimicrobial detergent for a washing machine; and miscellaneous antimicrobial products such as an antimicrobial detergent for use in kitchen and an antimicrobial detergent for tableware. Such a product may be in the form of liquid, gel, or solid, which is prepared through incorporation of benzyloxyethanol or a combination of benzyloxyethanol and paraben into a carrier.

Examples of liquid carriers which may be employed include water; alcohols such as ethanol and isopropyl alcohol; glycols such as propylene glycol, dipropylene glycol, and polyethylene glycol; esters such as phthalic acid esters and adipic acid esters; hydrocarbon oils such as liquid paraffin, isoparaffin, and squalane; and silicone oils such as methylpolysiloxane. Benzyloxyethanol is dissolved or dispersed in such a liquid carrier. If desired, an auxiliary ingredient may be added to and dissolved in the resultant solution or dispersion. Examples of the auxiliary ingredient which may be employed include nonionic, anionic, and cationic surfactants, a coloring agent, a viscosity regulating agent, an antioxidant, and a pH adjusting agent.

Examples of gel carriers include agar, carrageenan, gelatin, carboxymethyl cellulose, paste, alginic acid, polyvinyl alcohol, dextrin, and metal soap gel. Benzyloxyethanol is incorporated into a gel carrier by means of, for example, a method in which benzyloxyethanol is mixed with and dispersed in a gel material which has been prepared in advance, or a method in which benzyloxyethanol is added to a raw material for producing a gel material during the course of production of the gel material.

Examples of solid carriers which may be employed include inorganic and organic porous materials. Examples of the inorganic porous material include diatomaceous earth, loess, clay, biscuit material, talc, smectite, montmorillonite, calcium carbonate, zeolite, attapulgite, sepiolite, silica, silica gel, alumina, magnesia, silica-alumina, silica-magnesia, and synthetic aluminosilicate. Examples of the organic porous material include activated carbon, paper (e.g., filter paper), cellulose products, wood products, potpourri (e.g., dried plant or flower), non-woven fabric, woven fabric, and dextrin. Such a porous material to be employed may be in the form of, for example, powder, beads, pellets, spherical material, columnar material, or cylindrical material. Benzyloxyethanol may be incorporated into a solid carrier other than the aforementioned carriers, such as a synthetic-resin-made sheet, fiber, or building material through impregnation, kneading, or a similar technique. In such a case, benzyloxyethanol (i.e., active ingredient) may be incorporated into a porous material or encapsulated in a microcapsule in advance.

The aforementioned product may contain another antibacterial or antifungal agent in combination with benzyloxyethanol, so as to enhance the antibacterial or/and antifungal activity of the product. Specific examples of the agent include antifungal agents such as imidazole compounds; antimicrobial agents such as parabens; natural antimicrobial agents such as Moso bamboo extract; and antimicrobial agents exhibiting bleaching effects, such as hypochlorites and hydrogen peroxide. In this case, preferably, a paraben is employed in combination with benzyloxyethanol, from the viewpoint of further enhancement of the antimicrobial activity of the product.

The aforementioned product may further contain a generally employed perfume, so as to improve a sensation upon use of the product, or to mask a bacterial or fungal odor.

The aforementioned product produced through incorporation of benzyloxyethanol into a liquid carrier may be in the form of a trigger-spray-type product, a squeeze-type product having an opening, a bottle-type product having a measuring cap, an aerosol-spray-type product, a mechanical-spray-type product, a capsule-type product, or a viscosity-regulated-gel-type product.

The amount of benzyloxyethanol contained in any of the aforementioned household or miscellaneous products is 0.1 to 2 mass%, much more preferably 0.1 to 1 mass%, of the composition of the product. In the case where a paraben is employed in combination with benzyloxyethanol, the amount of the paraben contained in the product is regulated to 0.02 to 0.5 mass%, preferably 0.05 to 0.4 mass%, of the composition.

### Examples

### Test Example 1 Benzyloxyethanol sensitization test

Guinea pig maximization test was performed. Specifically, groups of guinea pigs, each group consisting of 10 guinea pigs, were employed, and each animal was given interedermal injection of.0.3(W/V)% benzyloxyethanol and 30(W/V)% benzyloxyethanol was applied occlusive patch. Subsequently, challenge was performed at a concentration of benzyloxyethanol at which no irritation occurs. When erythema was observed at the reacted area on the skin, the sensitivity was evaluated as "positive" for benzyloxyethanol. As a result, the positive rate was found to be 0%.

### Example 1 Antimicrobial activity of benzyloxyethanol

The minimum inhibitory concentration (MIC) of benzyloxyethanol against bacteria and fungi was determined by means of the agar plate method. The results are shown below.

There were employed the following microbial strains: *Staphylococcus aureus* IFO 13276, *Escherichia coli* IFO 3972, *Pseudomonas aeruginosa* IFO 13275, and *Alcaligenes faecalis* IFO 13111, and the following fungal strains: *Penicillium* citrinum IFO 6352 , *Cladosporium cladosporoides* IFO 6348 and *Aspergillus* niger IFO 6341. The media used in the test wasSCD agar medium (product of Wako Pure Chemical Industries, Ltd.) for the bacteria, and GP agar medium (product of Wako Pure Chemical Industries, Ltd.) for the fungi, to which various concentration of benzyloxyethanol was added.

**Table 1**

| MIC (%) against bacteria and fungi | |
|---|---|
| Strain | MIC (%) |
| *Staphylococcus aureus* IFO 13276 | 1.4 |
| *Escherichia coli* IFO 3972 | 0.8 |
| *Pseudomonas aeruginosa* IFO 13275 | 1.0 |
| *Alcaligenes faecalis* IFO 13111 | 0.8 |
| *Cladosporium cladosporoides* IFO 6348 | 0.2 |
| *Penicillium citrinum* IFO 6352 | 0.8 |
| *Aspergillus niger* IFO 6341 | 0.8 |

### Example 2 Synergistic effect of benzyloxyethanol (BOE) and methyl paraben on preservative efficacy

Preservative efficacy test was performed at 30°C by use of the bacteria written below and a sample (50 g) having a composition shown in the below-described Table 2, and the preservative efficacy was evaluated on the reduction in count of viable bacteria.

The following test microorganisms are challenged: *Staphylococcus aureus* IFO 13276, *Escherichia coli* IFO 3972, and *Pseudomonas aeruginosa* IFO 13275. The results of the test are shown in Table 2.

**Table 2**

| (Mass%) | | | |
|---|---|---|---|
| | A | B | C |
| Succinic acid | 0.040 | 0.040 | 0.040 |
| Potassium hydroxide | 0.038 | 0.038 | 0.038 |
| BOE | 0.5 | 0 | 0.3 |
| Methyl paraben | 0 | 0.2 | 0.05 |
| Purified water | Balance | Balance | Balance |
| Time required for the reduction (on the order of 10⁴) in count of viable bacteria (days) | 3 | 3 | 3 |

When BOE and methyl paraben were employed in combination, the available amounts of BOE and methyl paraben for the required reduction were reduced by 40% and 75%, respectively; i.e., the synergistic effect of these compounds was observed.

### Example 3 Formulations

### Formulation 1 Lotion

**Table 3**

| | (Mass%) |
|---|---|
| 1. Polyoxyethylene octyldodecyl ether | 0.4 |
| 2. Glycerin | 5.0 |
| 3. Dipropylene glycol | 2.0 |
| 4. Polyvinyl pyrrolidone | 0.1 |
| 5. Succinic acid | 0.08 |
| 6. Potassium hydroxide | 0.032 |
| 7. *Thujopsis dolabrata* extract | 0.5 |
| 8. Methyl p-hydroxybenzoate | 0.1 |
| 9. Benzyloxyethanol | 0.3 |
| 10. Purified water | Balance |

### (Preparation process and preservative efficacy)

Ingredients other than ingredient 7 were uniformly mixed together under heating, and the resultant aqueous solution was cooled to room temperature. Subsequently, ingredient 7 was added to the resultant solution, followed by stirring, to thereby produce a lotion. The lotion exhibited strong preservative efficacy.

### Reference Formulation 2 Lotion

**Table 4**

| | (Mass%) |
|---|---|
| 1. Polyoxyethylene hydrogenated castor oil | 0.4 |
| 2. Glycerin | 4.0 |
| 3. 1,3-Butylene glycol | 3.0 |
| 4. Trimethylglycine | 3.0 |
| 5. Pullulan | 0.2 |
| 6. Succinic acid | 0.08 |
| 7. Potassium hydroxide | 0.032 |
| 8. Eucalyptus extract | 0.5 |
| 9. Benzyloxyethanol | 0.9 |
| 10. Purified water | Balance |

### (Preparation process and preservative efficacy)

Ingredients other than ingredient 8 were uniformly mixed together under heating, and the resultant aqueous solution was cooled to room temperature. Subsequently, ingredient 8 was added to the resultant solution, followed by stirring, to thereby produce a lotion. The lotion exhibited strong preservative efficacy.

### Reference Formulation 3 Lotion

**Table 5**

| | (Mass%) |
|---|---|
| 1. Polyoxyethylene isocetyl ether | 0.3 |
| 2. Glycerin | 5.0 |
| 3. Propylene glycol | 2.0 |
| 4. Succinic acid | 0.05 |
| 5. Sodium monohydrogen phosphate | 0.25 |
| 6. Citrus junos extract | 0.5 |
| 7. Benzyloxyethanol | 0.4 |
| 8. Phenoxyethanol | 0.4 |
| 9. Purified water | Balance |

### (Preparation process and preseravative efficacy)

Ingredients other than ingredient 6 were uniformly mixed together under heating, and the resultant aqueous solution was cooled to room temperature. Subsequently, ingredient 6 was added to the resultant solution, followed by stirring, to thereby produce a lotion. The lotion exhibited strong preservative efficacy.

### Formulation 4 Emulsion

**Table 6**

| | (Mass%) |
|---|---|
| 1. Polyoxyethylene lauryl ether sodium phosphate | 0.3 |
| 2. Sorbitan monostearate | 0.3 |
| 3. N-stearoyl-N-methyltaurine sodium salt | 1.0 |
| 4. N-(hexadecyloxyhydroxypropyl)-N-hydroxyethylhexadecanamide | 2.0 |
| 5. Olive oil | 2.0 |
| 6. Squalane | 4.0 |
| 7. Cetyl alcohol | 0.3 |
| 8. Stearyl alcohol | 0.2 ' |
| 9. Methylpolysiloxane (6 cs) | 6.0 |
| 10. Carboxyvinyl polymer | 0.2 |
| 11. Glycerin | 10.0 |
| 12. Succinic acid | 0.01 |
| 13. Potassium hydroxide | 0.07 |
| 14. Methyl p-hydroxybenzoate | 0.15 |
| 15. Benzyloxyethanol | 0.4 |
| 16. Purified water | Balance |

### (Preparation process and preservative activity)

Ingredient 10 was dispersed in ingredient 9 at room temperature, and the resultant dispersion was added to an aqueous solution prepared by uniformly mixing ingredients 11 through 16 together under heating, to thereby completely dissolve the ingredient 10 in the solution. The resultant solution was gradually added to a solution prepared from ingredients 1 through 8 under heating, and the resultant mixture was stirred by use of a homo-mixer, followed by cooling to room temperature, to thereby produce an emulsion. The emulsion exhibited strong preservative activity.

### Reference Formulation 5 Emulsion

**Table 7**

| | (Mass%) |
|---|---|
| 1. N-(2-hydroxy-3-hexadecyloxypropyl)-N-2-hydroxyethylhexadecanamide | 5.0 |
| 2. Stearic acid | 2.0 |
| 3. Cholesterol isostearate | 3.0 |
| 4. Cetyl alcohol | 3.0 |
| 5. Diglyceryl diisostearate | 10.0 |
| 6. Pentaglyceryl monostearate | 2.5 |
| 7. Dipropylene glycol | 3.0 |
| 8. Xanthan gum | 0.3 |
| 9. Thujopsis dolabrata extract | 1.0 |
| 10. Perfume | Appropriate amount |
| 11. Phenoxyethanol | 0.4 |
| 12. Benzyloxyethanol | 0.6 |
| 13. Purified water | Balance |

### (Preparation process and preservative efficacy)

Ingredients 1 through 6 were mixed together under heating, and an aqueous solution prepared by uniformly mixing ingredients 7, 8, 11, 12, and 13 together under heating was gradually added to the resultant mixture, followed by stirring by use of a homo-mixer. After the resultant mixture was cooled to room temperature, ingredients 9 and 10 were added to the mixture, followed by stirring, to thereby produce an emulsion. The emulsion exhibited strong preservative efficacy.

### Formulation 6 Cream

**Table 8**

| | (Mass%) |
|---|---|
| 1. Squalane | 10.0 |
| 2. Ceramide 2 | 0.01 |
| 3. Octyldodecyl myristate | 6.0 |
| 4. Cetyl alcohol | 8.0 |
| 5. POE (20) sorbitan monostearate | 2.0 |
| 6. Olive oil | 0.1 |
| 7. Dipropylene glycol | 6.0 |
| 8. Glycerin | 5.0 |
| 9. Fucus extract | 1.0 |
| 10. Succinic acid | 0.01 |
| 11. Methyl p-hydroxybenzoate | 0.2 |
| 12. Ethyl p-hydroxybenzoate | 0.05 |
| 13. Benzyloxyethanol | 0.3 |
| 14. Purified water | Balance |

### (Preparation process and preservative efficacy)

Ingredients 1 through 6 were mixed together under heating, and an aqueous solution prepared by uniformly mixing ingredients 7, 8, and 10 through 14 together under heating was gradually added to the resultant mixture, followed by stirring by use of a homo-mixer. After the resultant mixture was cooled to room temperature, ingredient 9 was added to the mixture, followed by stirring, to thereby produce a cream. The cream exhibited strong preservative efficacy.

### Reference Formulation 7 Beauty lotion

**Table 9**

| | (Mass%) |
|---|---|
| 1. Hydroxyethyl cellulose | 0.5 |
| 2. Propylene glycol | 4.0 |
| 3. Glycerin | 5.0 |
| 4. Succinic acid | 0.05 |
| 5. Sodium monohydrogen phosphate | 0.25 |
| 6. Citrus junos extract | 0.5 |
| 7. Thujopsis dolabrata extract | 1.0 |
| 8. Fucus extract | 0.3 |
| 9. Phenoxyethanol | 0.5 |
| 10. Benzyloxyethanol | 0.5 |
| 11. Purified water | Balance |

### (Preparation process and preservative efficacy)

Ingredient 1 was dispersed in ingredient 2, and the resultant dispersion was added to a solution prepared through mixing of ingredients 3 through 5 and 9 through 11 and was uniformly dissolved in the solution under heating. After the resultant solution was cooled to room temperature, ingredients 6 through 8 were added to the solution, followed by stirring, to thereby produce a beauty lotion. The beauty lotion exhibited strong preservative efficacy.

### Formulation 8 Pack

**Table 10**

| | (Mass%) |
|---|---|
| 1. Polyvinyl alcohol | 12.0 |
| 2. Polyoxyethylene hydrogenated castor oil | 1.0 |
| 3. Glyceryl monoisostearate monomyristate | 1.0 |
| 4. Glycerin | 2.0 |
| 5. Pullulan | 1.0 |
| 6. Xanthan gum | 0.2 |
| 7. Methyl p-hydroxybenzoate | 0.15 |
| 8. Benzyloxyethanol | 0.3 |
| 9. Purified water | Balance |

### (Preparation process and preservative efficacy)

An aqueous solution prepared by uniformly mixing ingredients 4 and 7 through 9 together under heating was gradually added to a solution prepared by uniformly mixing ingredients 2 and 3 together under heating, followed by stirring by use of a homo-mixer. Ingredients 1, 5, and 6 were added to the resultant solution, and these ingredients were uniformly dissolved in the solution under heating, followed by cooling to room temperature, to thereby produce a pack. The pack exhibited strong preservative efficacy.

## Claims

1. A composition of an external dermatological agent comprising benzyloxyethanol and a preservative, wherein the content of benzyloxyethanol is 0.1 to 2 mass-% and said preservative is a paraben in an amount between 0.02 and 0.5 mass-Y, and wherein the following composition is excluded:
| | | |
|---|---|---|
| N,N-Dimethyloctadecyloxypropylamine | | 2.4 mass-% |
| Stearyl alcohol | | 7.2 mass-% |
| Propylene glycol | | 2.0 mass-% |
| Amphiphatic amide lipid A | | 0.5 mass-% |
| Sunflower oil | | 0.9 mass-% |
| Camellia oil | | 0.2 mass-% |
| Cholesteryl hydroxystearate | | 0.1 mass-% |
| Dipentaerythrol fatty acid ester . | | 0.3 mass-% |
| | (fatty acids: hydroxystearic acid, stearic acid and rosin acid) | |
| Benzyloxyethanol | | 0.5 mass-% |
| Methylpolysiloxane (100000 mPa*s) | | 3.7 mass-% |
| Aqueous solution of lactic acid (90 mass-%) | | 0.9 mass-% |
| Methylparaben | | 0.1 mass-% |
| Perfume | | 0.3 mass-% |
| Purified water | | balance. |

2. Use of benzyloxyethanol for providing an object with antibacterial and antifungal activities.

3. Use of benzyloxyethanol for providing an object with preservative efficacy.

4. Use of benzyloxyethanol according to claim 2 or 3, wherein a paraben is used in combination therewith.

## Patentansprüche

1. Zusammensetzung für ein äußerliches dermatologisches Mittel, das Benzyloxyethanol und ein Konservierungsmittel umfaßt, wobei der Gehalt an Benzyloxyethanol 0,1 bis 2 Gew.% ist, und das Konservierungsmittel ein Paraben in einer Menge zwischen 0,02 und 0,5 Gew.% ist, wobei die folgende Zusammensetzung ausgenommen ist:
| | | |
|---|---|---|
| N,N-Dimethyloctadecyloxypropylamin | | 2,4 Gew.% |
| Stearylalkohol | | 7,2 Gew.% |
| Propylenglycol | | 2,0 Gew.% |
| amphiphatisches Amidlipid A | | 0,5 Gew.% |
| Sonnenblumenöl | | 0,9 Gew.% |
| Kameliaöl | | 0,2 Gew.% |
| Cholesterylhydroxystearat | | 0,1 Gew.% |
| Dipentaerythrol-Fettsäureester | | 0,3 Gew.% |
| | (Fettsäuren: Hydroxystearinsäure, | |
| | Stearinsäure und Harzsäure) | |
| Benzyloxyethanol | | 0,5 Gew.% |
| Methylpolysiloxan (100.000 mPa·s) | | 3,7 Gew.% |
| wäßrige Milchsäurelösung (90 Gew.%) | | 0,9 Gew.% |
| Methylparaben | | 0,1 Gew.% |
| Parfüm | | 0,3 Gew.% |
| gereinigtes Wasser | | Restbetrag |

2. Verwendung von Benzyloxyethanol für die Bereitstellung eines Gegenstands mit antibakterieller und antimykotischer Wirkung.

3. Verwendung von Benzyloxyethanol für die Bereitstellung eines Gegenstands mit konservierender Wirkung.

4. Verwendung von Benzyloxyethanol gemäß Anspruch 2 oder 3, wobei ein Paraben in Kombination mit diesem verwendet wird.

## Revendications

1. Composition d'un agent dermatologique externe comprenant du benzyloxyéthanol et un conservateur, la teneur en benzyloxyéthanol étant de 0,1 à 2 % en masse et ledit conservateur étant un parabène dans une quantité entre 0,02 et 0,5 % en masse, et dans lequel la composition suivante est exclue :
| | |
|---|---|
| N,N-diméthyloctadécyloxypropylamine | 2,4 % en masse |
| Alcool stéarylique | 7,2 % en masse |
| Propylène glycol | 2,0 % en masse |
| Lipide A amide amphiphatique | 0,5 % en masse |
| Huile de tournesol | 0,9 % en masse |
| Huile de camélia | 0,2 % en masse |
| Hydroxystéarate de cholestéryle | 0,1 % en masse |
| Ester d'acide gras dipentaérythrol | 0,3 % en masse |
| (acides gras : acide hydroxystéarique, acide stéarique et acide résinique) | |
| Benzyloxyéthanol | 0,5 % en masse |
| Méthylpolysiloxane (100 000 mPa*s) | 3,7 % en masse |
| Solution aqueuse d'acide lactique (90 % | |
| en masse) | 0,9 % en masse |
| Méthylparabène | 0,1 % en masse |
| Parfum | 0,3 % en masse |
| Eau purifiée | reste |

2. Utilisation de benzyloxyéthanol pour l'obtention d'un objet ayant des activités antibactériennes et antifongiques.

3. Utilisation de benzyloxyéthanol pour l'obtention d'un objet ayant une efficacité de conservateur.

4. Utilisation de benzyloxyéthanol selon la revendication 2 ou 3, dans laquelle un parabène est utilisé en combinaison avec celui-ci.
